# EUROPEAN PATENT APPLICATION

(11) **EP 4 721 676 A1**
(43) Date of publication of application: **08.04.2026**
(21) Application number: 24815627.5
(22) Date of filing: 31.05.2024
(51) Int. Cl.: A61B 8/14, A61B 8/08, G06T 7/00

(54) **IMAGE EVALUATION METHOD, IMAGE EVALUATION DEVICE, COMPUTER PROGRAM, AND NON-TRANSITORY COMPUTER-READABLE MEDIUM**

(30) Priority: 31.05.2023 JP 2023090404
(71) Applicant: Tokyo Denki University, Tokyo 120-8551 (JP); TOHOKU UNIVERSITY, Sendai-shi Miyagi 980-8577 (JP); Tokyo University of Science Foundation, Tokyo 162-8601 (JP)
(72) Inventor: KUWANA Kenta, Tokyo 120-8551 (JP); KATO Hirotaka, Tokyo 120-8551 (JP); YOSHIDA Mikako, Sendai-shi, Miyagi 980-8577 (JP); MORI Taketoshi, Tokyo 162-8601 (JP); TANAKA Roberto, Tokyo 120-8551 (JP)
(74) Representative: Mewburn Ellis LLP
(86) International application number: PCT/JP2024/020089
(87) International publication number: WO 2024/248147

(57) **Abstract**

It is acquired image data corresponding to an ultrasonic image in which a pelvic region of a subject is captured. Image processing is applied with respect to the image data to extract multiple feature points associated with a prescribed portion. It is calculated an index value associated with at least one of a lower urinary tract function and a pelvic floor function of the subject based on the feature points.

## Description

### Field

The presently disclosed subject matter relates to a switch system including a switch device and a control device.

### Background

Patent Document 1 discloses a device capable of acquiring an ultrasonic image in which a pelvic region of a subject is captured. The ultrasonic image may be used in, for example, evaluation of the lower urinary tract function after total prostatectomy, pelvic floor muscular training for suppressing incontinence caused by pelvic floor hypofunction that may occur after delivery, or the like. However, the evaluation of the ultrasonic image depends on the subject view or experience of the observer.

### Citation List

### Patent Document

Patent Document 1: Japanese Patent Publication No. 2018-504185A

### Summary of the Invention

### Technical Problem

It is desired to provide a technique for enabling objective evaluation of indices related to at least one of the lower urinary tract function and the pelvic floor function based on an ultrasonic image in which a pelvic region of a subject is captured.

### Solution to Problem

An illustrative aspect of the presently disclosed subject matter may provide an image evaluation method adapted to be executed by at least one computing device, the image evaluation method comprising:
acquiring image data corresponding to an ultrasonic image in which a pelvic region of a subject is captured;
applying image processing with respect to the image data to extract multiple feature points associated with a prescribed portion; and
calculate an index value associated with at least one of a lower urinary tract function and a pelvic floor function of the subject based on the feature points.

An illustrative aspect of the presently disclosed subject matter may provide an image evaluation device, comprising:
an interface configured to acquire image data corresponding to an ultrasonic image in which a pelvic region of a subject is captured; and
a processor configured to:
   apply image processing with respect to the image data to extract multiple feature points associated with a prescribed portion; and
   calculate an index value associated with at least one of a lower urinary tract function and a pelvic floor function of the subject based on the feature points.

An illustrative aspect of the presently disclosed subject matter may provide a computer program adapted to be executed by a processor installed in an image evaluation device, the computer program being configured to, when executed, to cause the image evaluation device to:
receive image data corresponding to an ultrasonic image in which a pelvic region of a subject is captured;
apply image processing with respect to the image data to extract multiple feature points associated with a prescribed portion; and
calculate an index value associated with at least one of a lower urinary tract function and a pelvic floor function of the subject based on the feature points.

An illustrative aspect of the presently disclosed subject matter may provide a non-transitory computer-readable medium having stored the above computer program.

According to the configuration of each of the above illustrative aspects, in response to input of an ultrasonic image in which a pelvic region of a subject is captured, the steps of processing until it is calculated an index value that is associated with at least one of the lower urinary tract function and the pelvic floor function of the subject can be automated. Since the index value is obtained regardless of the subjective view or experience of the person observing the ultrasonic image, it is possible to provide an objective evaluation environment.

### Brief Description of Drawings

FIG. 1 schematically illustrates organs included in a pelvic region of a male.
FIG. 2 illustrates a condition that a total prostatectomy has been performed.
FIG. 3 illustrates an image evaluation method according to an exemplary embodiment.
FIG. 4 illustrates a detailed flow of image processing of FIG. 3.
FIG. 5 illustrates an ultrasonic image in which a pelvic region of a subject is captured.
FIG. 6 illustrates a state that a region of interest is specified in the ultrasonic image of FIG. 5.
FIG. 7 illustrates a state that only the region of interest of FIG. 6 is extracted.
FIG. 8 illustrates an image obtained by applying binarization with respect to the image of FIG. 7.
FIG. 9 illustrates an image obtained by applying edge extraction with respect to the image of FIG. 8.
FIG. 10 illustrates an image obtained by applying Hough transform with respect to the image of FIG. 9.
FIG. 11 is a diagram for explaining processing for calculating an angle of a bladder neck.
FIG. 12 is a diagram for explaining processing for calculating the angle of the bladder neck.
FIG. 13 is a diagram for explaining processing for calculating the angle of the bladder neck.
FIG. 14 illustrates a state that the angle of the bladder neck is displayed.
FIG. 15 is a diagram for explaining an angle of a bulbar urethra.
FIG. 16 illustrates an ultrasonic image in which a urethra of a subject is captured.
FIG. 17 is a diagram for explaining processing for calculating the angle of the bulbar urethra.
FIG. 18 is a diagram for explaining processing for calculating the angle of the bulbar urethra.
FIG. 19 is a diagram for explaining processing for calculating the angle of the bulbar urethra.
FIG. 20 is a diagram for explaining distance between an inferior margin of a pubis and an anorectal angle.
FIG. 21 is a diagram for explaining processing for calculating the distance between the inferior margin of the pubis and the anorectal angle.
FIG. 22 illustrates a functional configuration of an image evaluation device according to an exemplary embodiment.

### Description of Embodiments

With reference to the accompanying drawings, there will be described below in detail an exemplary embodiment of a method of evaluating indices related to at least one of the lower urinary tract function and the pelvic floor function based on an ultrasonic image in which a pelvic region of a subject is captured.

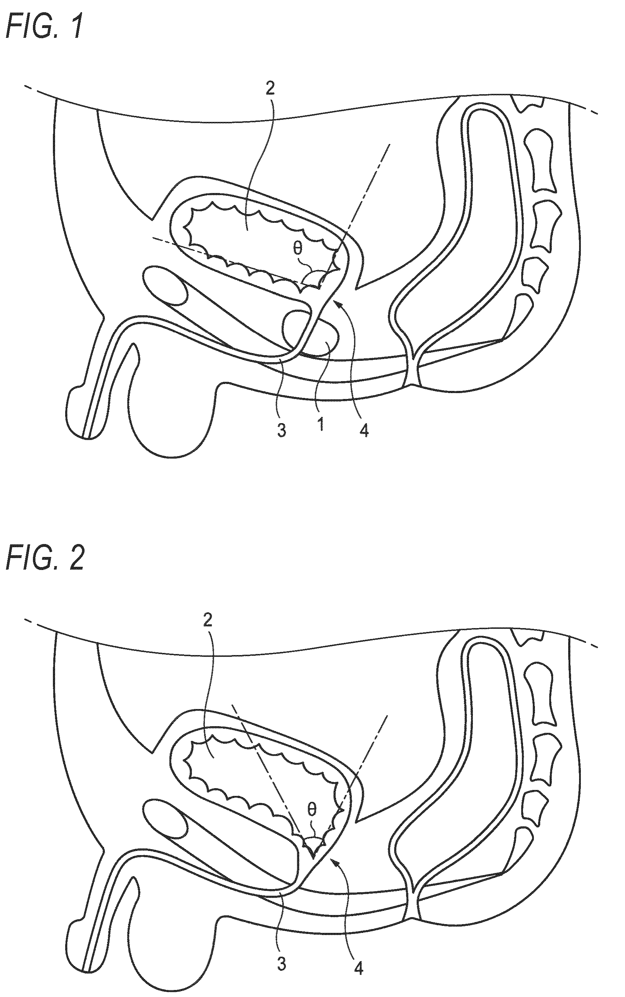

FIG. 1 schematically illustrates organs included in a pelvic region of a male. FIG. 2 illustrates a condition that a total prostatectomy known as radical treatment of prostate cancer has been performed.

In the surgery, a prostate gland 1 is excised. Then a bladder 2 and a urethra 3 are then clamped together. As a result of the surgery, the bladder 2 is pulled downward, so that funneling in which a bladder neck 4 is enlarged may occur. It is pointed out that the occurrence of funneling may contribute to the mechanism of urinary incontinence because the urine in the bladder 2 easily flows into the urethra 3 due to the enlargement. The urinary incontinence is an example of a disease associated with hypofunction of the lower urinary tract.

The occurrence of funneling is characterized by a fact that an angle θ of the bladder neck is less than a preoperative angle (180 degrees). In a case where it is recognized that the funneling occurs (in a case where a funneling angle is non-zero), the angle θ of the bladder neck corresponds to the degree of the funneling. In other words, higher severity of hypofunction of the lower urinary tract is estimated in a case where a larger degree of funneling (a larger angle θ) is observed. Medical workers visually confirm an ultrasonic image in which the pelvic region of the subject is captured to evaluate the lower urinary tract function.

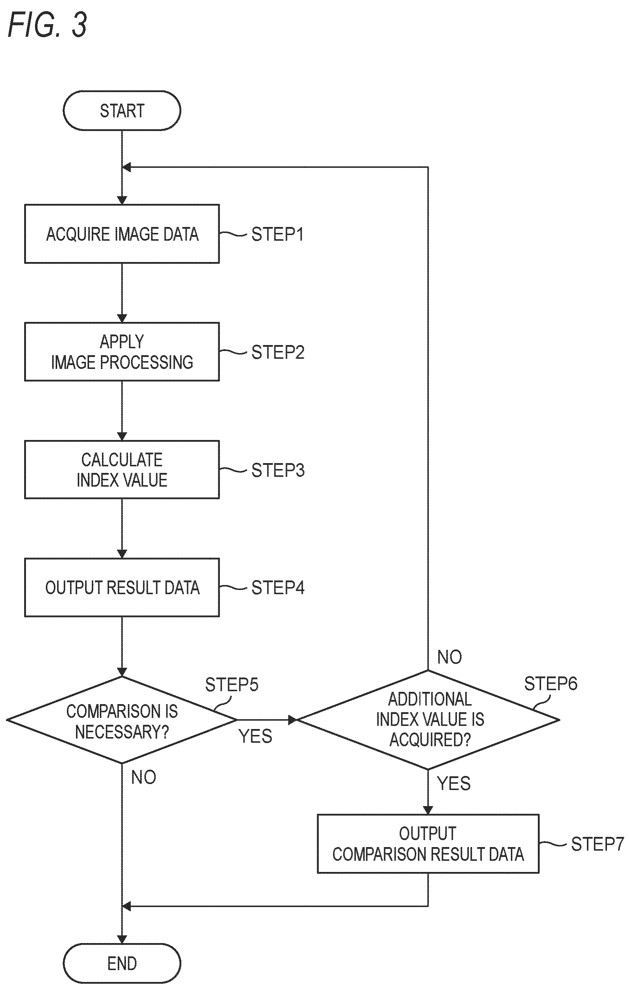

An image evaluation method according to an exemplary embodiment will be described with reference to FIG. 3. This method is based on an idea that the angle θ of the bladder neck may be an index value for quantitatively evaluating the lower urinary tract function. In this method, such an index value is automatically calculated based on the ultrasonic image in which the pelvic region of the subject is captured.

First, it is acquired image data corresponding to an ultrasonic image in which a pelvic region of a subject is captured (STEP 1). The ultrasonic image may be acquired by applying an ultrasonic probe to, for example, a crotch of the subject.

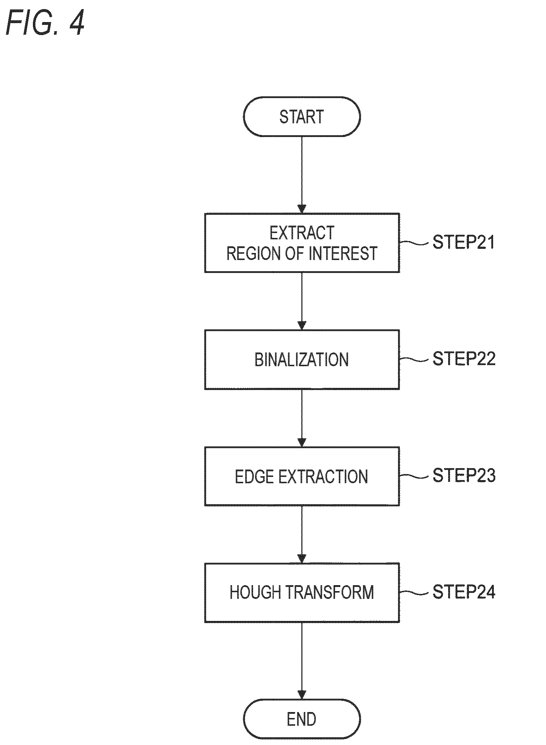

Subsequently, image processing is performed on the image data (STEP 2). The image processing is preliminarily performed for enabling the automatic calculation of the angle θ of the bladder neck. The detailed flow of the image processing will be described with reference to FIG. 4.

First, it is performed processing for extracting a region including at least the bladder neck, as a region of interest, from an ultrasonic image in which the pelvic region is captured (STEP 21). The processing is performed by applying a well-known object detection algorithm to the image data acquired in STEP 1.

In the present exemplary embodiment, YOLO (You Only Look Once) is used as the object detection algorithm. A region where the YOLO determines that the bladder neck is captured with a higher probability than a prescribed threshold is specified as the region of interest. For creating a deep learning model serving as a basis of the YOLO, what is used is training data in which an annotation is assigned with respect to a position of the bladder neck in the ultrasonic image in which the pelvic region is captured.

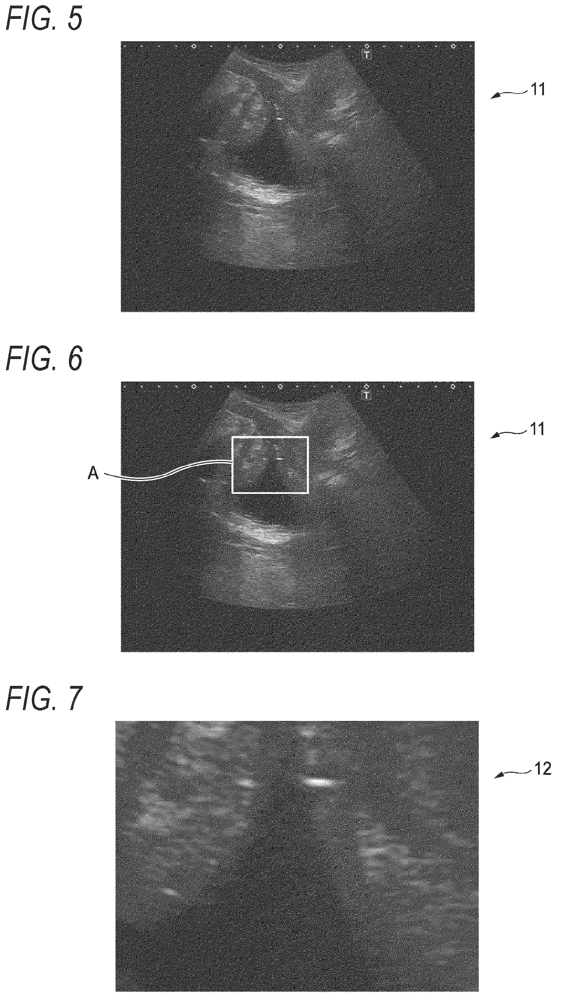

FIG. 5 illustrates an ultrasonic image 11 in which a pelvic region of a subject is captured. It should be noted that the up-down direction in this image is opposite to that of FIG. 2. In other words, the bladder neck appears in the upper portion of the bladder. FIG. 6 illustrates a state where a region of interest A is specified in the ultrasonic image 11 by applying the YOLO. FIG. 7 illustrates an extracted image 12 in which only the region of interest A is extracted from the ultrasonic image 11.

Subsequently, binarization is performed with respect to the extracted image 12 (STEP 22 in FIG. 4). Each of multiple pixels constituting the extracted image 12 has any one of gradation values the number of which is capable of expressing shades of gray. In the binarization, the gradation value of each pixel is converted to one of a value corresponding to white and a value corresponding to black.

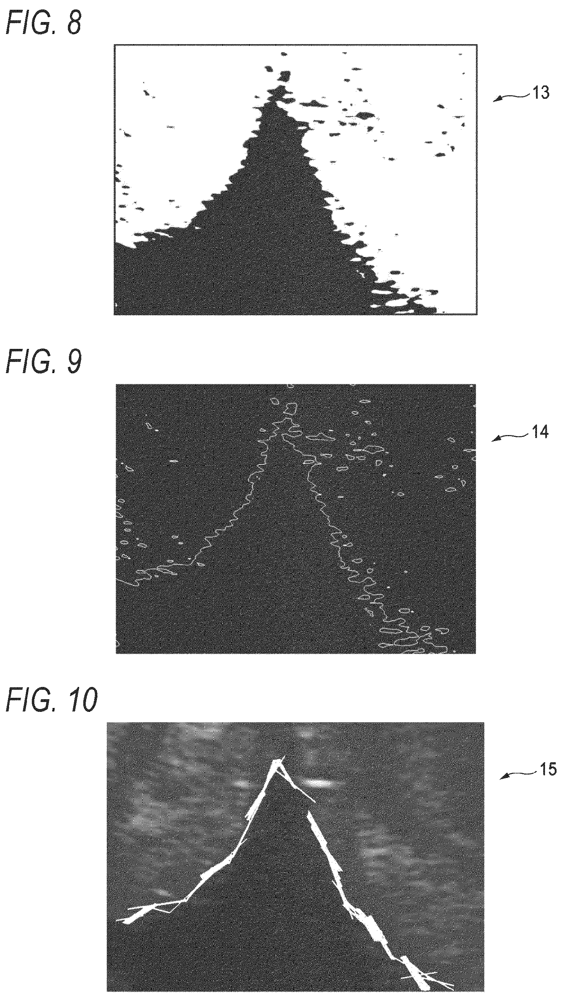

For example, in a case where the gradation value of a certain pixel is less than a threshold, the gradation value is converted to the value corresponding to black, whereas in a case where the gradation value is no less than the threshold, the gradation value is converted to the value corresponding to white. The threshold value may be a fixed value that is determined in advance, or may be a variable value that is determined through the discriminant analysis method. FIG. 8 illustrates a binarized image 13 that is obtained by applying the binarization with respect to the extracted image 12.

Subsequently, edge extraction is performed with respect to the binarized image 13 (STEP 23 in FIG. 4). The edge extraction is processing for emphasizing a part where a difference between the gradation values of adjacent pixels is large. As is apparent from the binarized image 13, the gradation value largely changes at a boundary portion between a lumen and an inner wall of the bladder. Accordingly, the boundary portion constituting a part of the bladder neck is emphasized by the edge extraction.

As a technique for performing the edge extraction, the Sobel method, the Laplacian method, or the like is known. In this example, the Laplacian method is used because edges can be smoothly extracted while suppressing the influence of noise. FIG. 9 illustrates an edge extraction image 14 that is obtained by applying the edge extraction with respect to the binarized image 13.

Subsequently, a Hough transform is performed with respect to the edge extraction image 14 (STEP 24 in FIG. 4). The Hough transform is processing for detecting a linear line in the image as inputted. FIG. 10 illustrates a linear detection image 15 that is obtained by applying the Hough transform with respect to the edge extraction image 14. A number of linear lines are displayed which are arranged along the boundary portion between the lumen and the inner wall of the bladder.

It should be noted that at least the binarized image 13, the edge extraction image 14, and the linear detection image 15 are illustrated for the sake of convenience of understanding, and may not be visualized to the user. Each processing may be described as conversion processing to be applied with respect to the data corresponding to each image without involving visualization.

As illustrated in FIG. 3, calculation of the index value is subsequently performed (STEP 3). Based on the linear detection image 15 described above, the angle θ of the bladder neck of the subject is calculated as the index value.

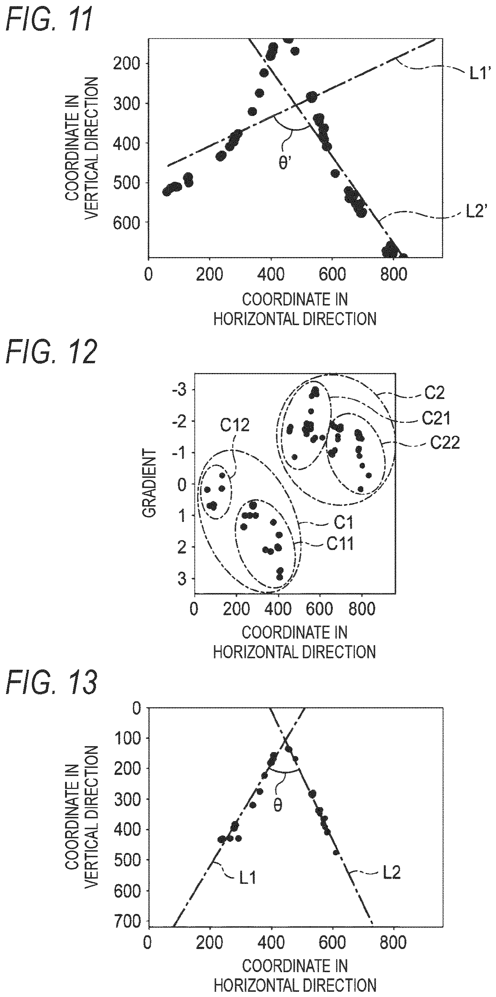

FIG. 11 illustrates a graph that is obtained by plotting representative points each of which corresponds to a midpoint of each of the linear lines that are detected in the linear detection image 15. The abscissa represents a coordinate in a horizontal direction in the linear detection image 15, and the ordinate represents a coordinate in a vertical direction in the same image.

Subsequently, clustering is applied to with respect to the large number of plots obtained as described above. Specifically, a gradient value of the linear line that is the basis of each plot in the linear detection image 15 is referred to. As illustrated in FIG. 12, a plot obtained from a linear line the gradient of which has a positive value is classified into a cluster C1. A plot obtained from a linear line the gradient of which has a negative value is classified into a cluster C2. In FIG. 12, the abscissa represents a coordinate in the horizontal direction in the linear detection image 15, and the ordinate represents a gradient.

In addition, among the plots classified into the cluster C1, a plot an absolute value of the gradient of which is no less than a threshold is classified into a cluster C11, whereas a plot the absolute value of the gradient of which is less than the threshold is classified into a cluster C12. Similarly, among the plots classified into the cluster C2, a plot an absolute value of the gradient of which is no less than a threshold is classified into a cluster C21, whereas a plot the absolute value of the gradient of which is less than the threshold is classified into a cluster C22.

FIG. 13 illustrates a state where the plots classified into the cluster C12 and the cluster C22 are eliminated from the plots illustrated in FIG. 11. In other words, the plots the absolute gradient value of which is no less than the threshold are left.

A linear regression is applied with respect to the plots belonging to the cluster C12 after being weighted in accordance with the length of the linear line that is obtained by applying the Hough transform, thereby obtaining a linear line L1. The linear line L1 corresponds to a linear line approximating an inner wall face of the bladder neck appearing in the edge extraction image 12 of FIG. 5 so as to have a positive gradient. Similarly, the linear regression is applied to the plots belonging to the cluster C22, thereby obtaining a linear line L2. The linear line L1 corresponds to a linear line approximating an inner wall face of the bladder neck appearing in the edge extraction image 12 of FIG. 5 so as to have a negative gradient. The angle θ formed by the linear line L1 and the linear line L2 is specified as the angle of the bladder neck.

The above-described clustering policy in which not only the positive or negative of the gradient, but also the absolute value of the gradient are considered is based on the finding that the bladder neck involving the funneling has a shape including a portion that has a relatively large gradient and a portion that has a relatively small gradient.

The linear line L1' illustrated in FIG. 11 is obtained by applying the linear regression with respect to all the plots belonging to the cluster C1. Similarly, the linear line L2' is obtained by applying linear regression with respect to all the plots belonging to the cluster C2. These linear lines may not adequately approximate the shape of the actual bladder neck. Accordingly, an angle θ' formed by the linear lines L1' and L2' may not correspond to the angle of the actual bladder neck.

As can be seen from the comparison between FIGS. 11 and 13, a value closer to the actual angle of the bladder neck can be calculated with the processing that focuses on a portion of the bladder neck that has a relatively large gradient.

The above-described clustering may be realized by applying a well-known technique at least once. Examples of such a well-known technique include the k-means method, the mixed Gaussian distribution, and the like. It should be noted that the clustering may not be performed so as to focus on both the negative/positive and the absolute value of the gradient of the linear line. For example, at least one clustering may be performed so as to focus on the absolute value of the gradient only.

As illustrated in FIG. 3, processing for outputting result data is subsequently performed (STEP 4). The result data is configured to include at least data corresponding to the angle of the bladder neck that is calculated as described above.

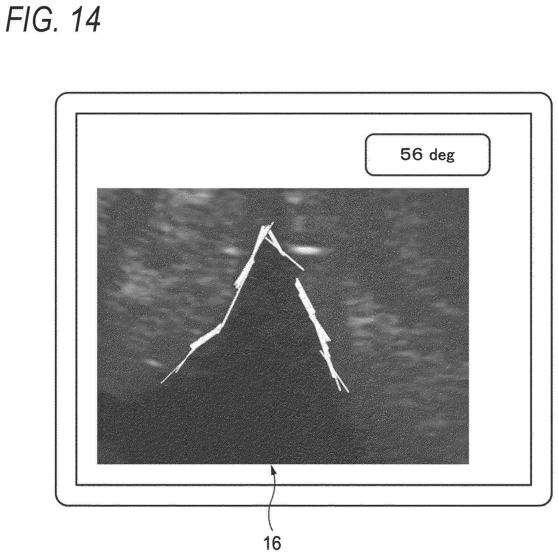

FIG. 14 illustrates an exemplary way of outputting the result data. In this example, the angle of the bladder neck as calculated is displayed on a display device. In other words, the result data is configured to cause the display device to display the value of the angle. In this example, an image 16 that shall be the basis of the angle as calculated is also displayed on the display device. The image 16 according to the present example corresponds to an image in which linear lines that have no contribution to the calculation of the angle of the bladder neck are eliminated from the linear detection image 15 illustrated in FIG. 10.

The result data may be outputted by the way other than the displaying with the display device. The result data may be configured to adapt to a report form that is subjected to printing or data transmission.

As described above, according to the configuration of the present exemplary embodiment, in response to input of an ultrasonic image in which a pelvic region of a subject is captured, the steps of processing until the angle of the bladder neck of the subject is calculated can be automated. As a result, it is possible to provide an index value associated with the lower urinary tract function of the subject, regardless of the subjective view or experience of the person observing the ultrasonic image. In other words, it is possible to provide an environment that enables objective evaluation of the index value associated with the function of the lower urinary tract of the subject.

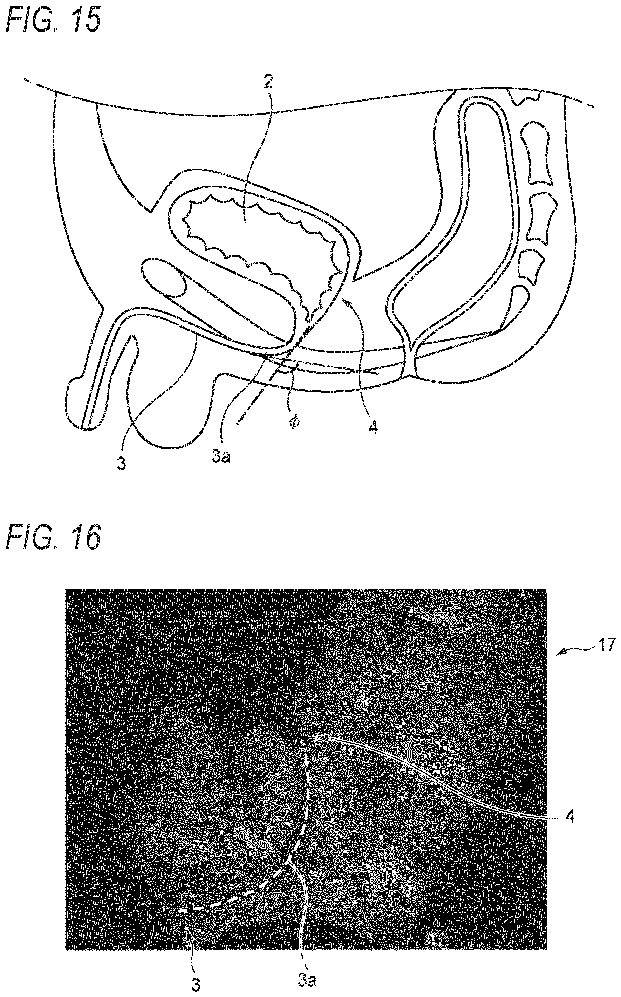

An angle of a bulbar urethra may be another exemplary index enabling the evaluation of the lower urinary tract function described above. As illustrated in FIG. 15, a bulbar urethra 3a is a portion of the urethra 3 that is significantly curved in the vicinity of the bladder neck 4. The angle φ of the bulbar urethra 3a is defined as an angle formed by two approximate lines extending along both ends of the bulbar urethra 3a.

FIG. 16 illustrates an ultrasonic image 17 corresponding to the image data acquired in STEP 1 of FIG. 3. The urethra 3 of the subject is captured in the ultrasonic image 17 (the position of the urethra 3 is highlighted with dashed lines). Since the urethra 3 is a very elongate tubular organ, it is not easy to visually discriminate the position of the urethra 3 in the ultrasonic image 17.

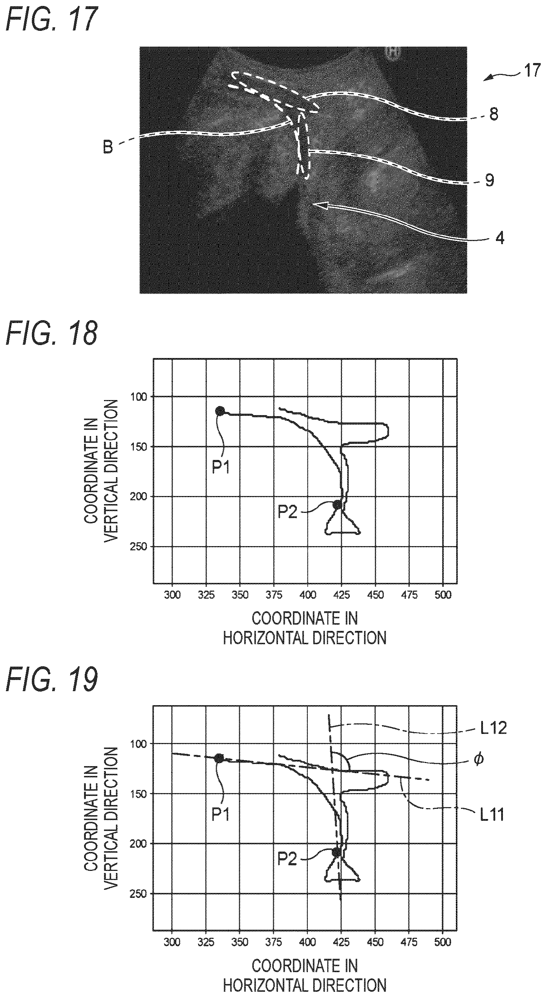

FIG. 17 shows the ultrasonic image 17 of FIG. 16 upside down. In other words, the urethra appears above the bladder neck 4. A bulbospongiosus muscle 8 and a soft tissue 9 that are adjacent to the urethra appear in the ultrasonic image 17 as a region with low brightness. Accordingly, a boundary B between the region with low brightness and an adjacent region with high brightness can be regarded as the urethra 3.

FIG. 18 illustrates a state that a contour of a region of interest corresponding to the region with low brightness and the region with high brightness is extracted by applying the image processing with respect to the image data corresponding to the ultrasonic image 17 (STEP 2 in FIG. 3).

In this example as well, the YOLO is adopted as the object detection algorithm. The YOLO determines a region where the possibility that the region of interest is captured exceeds a prescribed threshold. A contour of such a region is extracted. The contour is acquired as a set of points. For creating a deep learning model serving as a basis of the YOLO, what is used is training data in which an annotation is assigned so as to trace the contour with reference to a marking image of the region of interest prepared by an expert.

Subsequently, the angle of the bulbar urethra as the index value is calculated (STEP 3 in FIG. 3). Specifically, in order to trim a portion corresponding to the bulbar urethra 3a from the extracted contour, a first end point P1 and a second end point P2 are specified. The first end point P1 may be specified as, for example, a point that assumes an extremum in a case where the above-described points are regarded as a curve. As the second end point P2, for example, a coordinate of a point specified as the vertex of the bladder neck 4 may be used.

In FIG. 19, a first approximate linear line L11 and a second approximate linear line L12 are defined for the trimmed portion corresponding to the bulbar urethra 3a. The first approximation linear line L11 is defined by applying linear regression with respect to points in an adequate range including the first end point P1. The second approximation linear line L12 is defined by applying linear regression with respect to points in an adequate range including the second end point P2. The angle φ formed by the first approximation linear line L11 and the second approximation linear line L12 is specified as the angle of the bulbar urethra.

Subsequently, the result data is outputted (STEP 4 in FIG. 3). The result data according to the present example is configured to include at least the data corresponding to the angle φ that is calculated as described above. In addition to or in place of the displaying with the display device as described with reference to FIG. 14, the result data may be outputted by printing or data transmission of a report.

According to the configuration of the present exemplary embodiment, in response to input of an ultrasonic image in which a pelvic region of a subject is captured, the steps of processing until the angle of the bulbar urethra of the subject is calculated can be automated. As a result, it is possible to provide an index value associated with the lower urinary tract function of the subject, regardless of the subjective view or experience of the person observing the ultrasonic image. In other words, it is possible to provide an environment that enables objective evaluation of the index value associated with the function of the lower urinary tract of the subject.

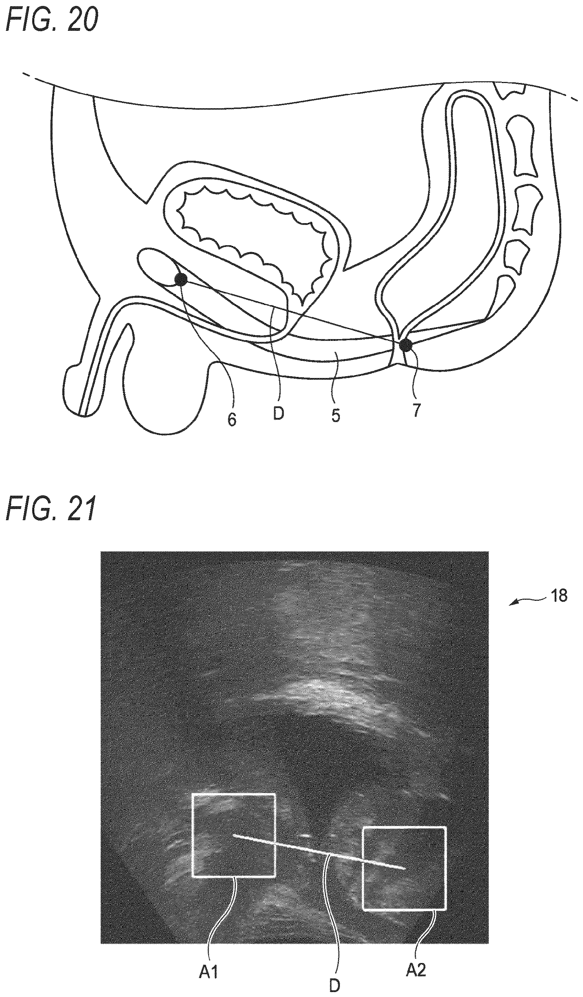

The pelvic floor muscles 5 illustrated in FIG. 20 are muscles forming a pelvic floor. The pelvic floor is a term comprehensively designating muscles, fascia, and ligaments that support pelvic organs (e.g., a uterus, a bladder, and a rectum). Training is performed to strengthen muscular strength of the pelvic floor muscles 5 in order to suppress incontinence caused by hypofunction of the pelvic floor that may occur after delivery, recover the lower urinary tract function that is depressed by the above-described total prostatectomy, and the like. In the following description, the training is referred to as "pelvic floor muscular training".

The pelvic floor muscular training is performed by causing a subject to consciously contract the pelvic floor muscles 5. As the pelvic floor muscles 5 are contracted, a distance D between an inferior margin of a pubis 6 and an anorectal angle 7 is decreased. The medical worker visually confirms the distance D in the ultrasonic image in which the pelvic region of the subject is captured, thereby evaluating the ability to contract the pelvic floor muscles 5 appropriately. In other words, the distance D may be an index value that quantitatively evaluates the function of the pelvic floor.

The image evaluation method described with reference to FIG. 3 may be likewise used for calculating such an index value. In other words, the distance D between the inferior margin of the pubis 6 and the anorectal angle 7 may be automatically calculated based on the ultrasonic image in which the pelvic region of the subject is captured.

FIG. 21 illustrates an ultrasonic image 18 corresponding to the image data acquired in STEP 1 of FIG. 3. The pelvic region of the subject is captured in the ultrasonic image 18. As the image data is subjected to image processing (STEP2 in FIG. 3), a region including at least the inferior margin of the pubis and a region including at least the anorectal angle are extracted as a region of interest.

In this example as well, the YOLO is adopted as the object detection algorithm. A region where the YOLO determines that the inferior margin of the pubis is captured with a higher probability than a prescribed threshold is specified as a region of interest A1. Similarly, a region where the YOLO determines that the anorectal angle is captured with a higher probability than a prescribed threshold is specified as a region of interest A2. For creating a deep learning model serving as a basis of the YOLO according to the present example, what is used is training data in which an annotation is assigned with respect to positions of the inferior margin of the pubis and the anorectal angle in the ultrasonic image in which the pelvic region is captured.

Subsequently, the distance D as the index value is calculated (STEP 3 in FIG. 3). In this example, a quantity corresponding to a length of a linear line connecting a central point of the region of interest A1 and a central point of the region of interest A2 is specified as the distance D. However, an adequate representative point may be determined for each region of interest, so that a quantity corresponding to a length of a linear line connecting such representative points may be specified as the distance D.

Subsequently, the result data is outputted (STEP 4 in FIG. 3). The result data according to the present example is configured to include at least data corresponding to the distance D that is calculated as described above. In addition to or in place of the displaying with the display device as described with reference to FIG. 14, the result data may be outputted by printing or data transmission of a report.

According to the configuration of the present exemplary embodiment, in response to input of an ultrasonic image in which a pelvic region of a subject is captured, the steps of processing until the distance between the inferior margin of the pubis and the anorectal angle of the subject is calculated can be automated. As a result, it is possible to provide an index value associated with the pelvic floor function of the subject, regardless of the subjective view or experience of the person observing the ultrasonic image. In other words, it is possible to provide an environment that enables objective evaluation of the index value associated with the function of the pelvic floor of the subject.

As described above, the pelvic floor muscular training is likewise performed for the purpose of restoring the lower urinary tract function. Accordingly, the distance between the inferior margin of the pubis and the anorectal angle may be an example of an index associated with the lower urinary tract function.

In a case where the above-described ultrasonic image 18 is acquired continuously over time, a temporal change of the distance D may be automatically acquired by processing for tracking the positions of the inferior margin of the pubis and the anorectal angle that are specified through the image processing. Examples of such processing include KCF (Kernelinzed Correction Filter), MIL (Multiple Instance Learning), TLD (Tracking Learning Detection), boosting, median flow, and the like.

As the temporal change of the distance D is acquired, it is possible to quantitatively evaluate index values such as contraction speed, contraction acceleration, contraction frequency, duration of the contracted condition, contraction repeatability, and delay from the muscle contraction command (volitional initiation of muscle contraction).

In addition, as illustrated in FIG. 21, by causing the display device to continuously display the ultrasonic image in which the region of interest is captured, the contracting state of the pelvic floor muscles is more clearly visualized. As a result, in the feedback to the subject of the pelvic floor muscular training, it is possible to facilitate understanding with respect to success/failure of exercise as well as how to improve the same.

The result data outputted in STEP 4 of FIG. 3 may include a result of estimation of severity related to the deterioration of at least one of the urinary tract lower function and the pelvic floor function based on the various index values that are calculated as described above.

For instance, higher severity of the hypofunction of the lower urinary tract is estimated in a case where a larger angle θ of the bladder neck is observed because the angle is increased with the degree of tunneling. In another case, higher severity of the hypofunction of the pelvic floor is estimated in a case where it is observed a smaller amount of change in the distance between the inferior margin of the pubis and the anorectal angle that is acquired during the pelvic floor muscular training.

The data indicating correspondence between the index value and the severity is stored in advance in a storage device in a table format or as a result of the machine learning. A computing device refers to the data in response to input of the index value, thereby automatically performing evaluation of the severity.

Result data to be outputted is configured to visualize a result of the severity estimation with at least one of character, color, and symbol.

According to the above configuration, it is possible to automate the processing until the severity of the hypofunction of at least one of the lower urinary tract and the pelvic floor of the subject based on the ultrasonic image in which the pelvic region of the subject is captured. As a result, it is possible to provide an evaluation environment regardless of the subjective view or experience of the observer of the ultrasonic image.

The result data outputted in STEP 4 of FIG. 3 may include a method of the pelvic floor muscular training as selected based on at least one of the various index values calculated as described above and the severity estimated as described above.

The policy of selecting the training method based on an evaluation result of the contraction capability of the pelvic floor muscle is generalized. Accordingly, it is possible to automate the selection of a training method suitable for the subject by defining the correspondence between evaluation result of the contraction capability of the pelvic floor muscle and at least one of the index value acquired as described above and the severity estimated as described above. The data indicating the correspondence is stored in advance in a storage device in a table format or as a result of the machine learning. A computing device refers to the data in response to input of at least one of the index value and the estimation result of the severity, thereby automatically performing selection of the training method.

Result data to be outputted is configured to visualize a selection result of the training method with at least one of character, color, and symbol.

According to the above configuration, it is possible to automate the processing until a pelvic floor muscular training method suitable for the subject is selected based on the ultrasonic image in which the pelvic region of the subject is captured. As a result, it is possible to provide a guidance environment regardless of the subjective view or experience of the observer of the ultrasonic image.

As illustrated in FIG. 3, the image evaluation method according to the presently disclosed subject matter may be configured to enable comparison between index values that are calculated at different time points.

Specifically, after the result data is outputted at a certain time point, it is determined whether the comparison of the index values is necessary (STEP 5). As it is determined that the comparison is unnecessary (NO in STEP 5), the processing according to the present method is terminated.

As it is determined that the comparison of the index values is necessary (YES in STEP 5), it is determined whether an additional index value to be used for the comparison has been acquired (STEP 6). As it is determined that no additional index value has been acquired (NO in STEP 6), the processing is returned to STEP 1, so that the processing for acquiring the additional index value is repeated.

As it is determined that the additional index value has been acquired (YES in STEP 6), comparison with the index value that is acquired earlier is performed, so that data indicating a comparison result is outputted (STEP 7). The comparison result may be presented in a form where the index values acquired at different time points are listed, or a new index value that is obtained based on the multiple index values may be presented. Examples of the new index value include a difference value, an average value, and the like.

According to the above configuration, it is possible to automate follow-up of at least one of the lower urinary tract function and the pelvic floor function of the subject, result evaluation of the pelvic floor muscular training, and the like. Accordingly, it is possible to provide an evaluation environment regardless of the subjective view or experience of the observer of the ultrasonic image.

The image evaluation method illustrated in FIG. 3 may be performed by at least one computing device. Each of the processing steps may be performed by an individual computing device, or multiple processing steps may be performed by a single computing device. In a case where the image evaluation method is performed by multiple computing devices, these devices may be disposed in remote places. In this case, data communication between the computing devices is performed over a public or private communication network. The communication network may be implemented by any of wired connection, wireless connection, and combinations thereof.

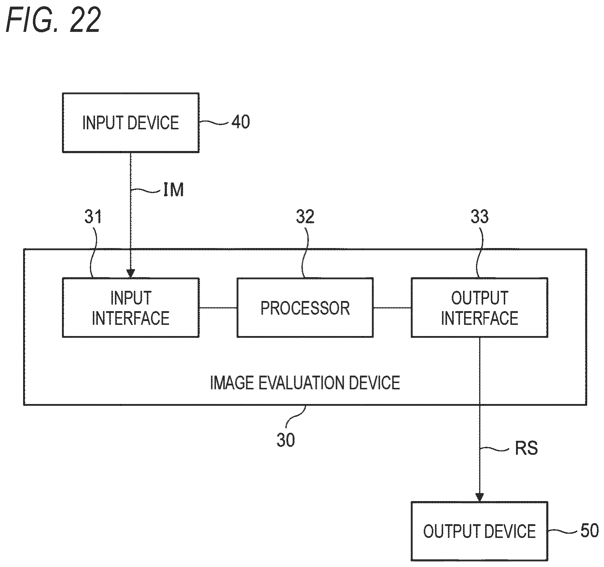

FIG. 22 illustrates a functional configuration of an image evaluation device 30 according to an exemplary embodiment. The image evaluation device 30 is an example of the computing device described above. The image evaluation device 30 may be a device that is disposed in a specific place, or a device that can be carried by a user.

The image evaluation device 30 includes an input interface 31, a processor 32, and an output interface 33.

The input interface 31 is configured as a hardware interface that receives image data IM from an input device 40. The image data IM corresponds to an ultrasonic image in which a pelvic region of a subject is captured. The input device 40 may be an ultrasonic probe that is applied to the body of the subject, or may be a device that visualizes the ultrasonic image. The image data IM may be in the form of analog data or digital data, in accordance with the specification of the input device 40. In a case where the image data IM is in the form of analog data, the input interface 31 is provided with an adequate conversion circuit including an A/D converter.

The processor 32 is configured to execute each of the processing steps described with reference to FIG. 3 based on the image data IM received by the input interface 31. The processor 32 having such a function may be implemented by at least one versatile microprocessor configured to cooperate with at least one versatile memory. Examples of the versatile microprocessor include a CPU, an MPU, and a GPU. Examples of the versatile memory include a ROM and a RAM. In this case, a computer program for implementing the function may be stored in the ROM. The versatile microprocessor designates at least a part of the program stored in the ROM, loads the designated program in the RAM, and executes the above-described processing in cooperation with the RAM. In this case, the versatile memory is an example of a non-transitory computer-readable medium having stored a computer program.

The processor 32 may be implemented by an exclusive integrated circuit provided with a memory element in which a computer program for implementing the function is pre-installed. Examples of the exclusive integrated circuit include a microcontroller, an ASIC, and an FPGA. In this case, the memory element is an example of a non-transitory computer-readable medium having stored a computer program. The processor 32 may be implemented by a combination of the versatile microprocessor and the exclusive integrated circuit.

The output interface 33 is configured as a hardware interface that outputs result data RS generated by the processor 32 to an output device 50. Examples of the output device 50 include a display device, a speaker, a printer, and a data transmission device. The output device 50 may be a part of the image evaluation device 30, or may be a device independent of the image evaluation device 30. The result data RS may be in the form of analog data or digital data, in accordance with the specification of the output device 50. In a case where the result data RS is in the form of analog data, the output interface 33 is provided with an adequate conversion circuit including a D/A converter.

Each of the configurations exemplified above is merely illustrative for facilitating understanding of the presently disclosed subject matter. Each exemplary configuration may be appropriately modified or combined with another exemplary configuration within the scope of the presently disclosed subject matter.

The index value associated with at least one of the lower urinary tract function and the pelvic floor function of the subject is not limited to the angle of the bladder neck, the distance between the inferior margin of the pubis and the anorectal angle, and the angle of the bulbar urethra. Another index value may be calculated as long as the value can be specified based on an ultrasonic image in which an organ included in a pelvic region of a subject is captured, and can be associated with at least one of the lower urinary tract function and the pelvic floor function. Examples of such an organ include bladder, uterus, urethra, vagina, and the like. Examples of such an index value include a moving amount of the bladder neck, a contraction rate of a levator ani hiatus area, and the like.

The present application is based on Japanese Patent Application No. 2023-090404 filed on May 31, 2023, the entire contents of which are incorporated herein by reference.

## Claims

1. An image evaluation method adapted to be executed by at least one computing device, the image evaluation method comprising:
acquiring image data corresponding to an ultrasonic image in which a pelvic region of a subject is captured;
applying image processing with respect to the image data to extract multiple feature points associated with a prescribed portion; and
calculate an index value associated with at least one of a lower urinary tract function and a pelvic floor function of the subject based on the feature points.

2. The image evaluation method according to claim 1,
wherein the feature points correspond to a part of a bladder neck; and
wherein the index value corresponds to an angle of the bladder neck.

3. The image evaluation method according to claim 2,
wherein each of the feature points is a part of a linear line that is extracted by the image processing; and
wherein clustering is performed so as to focus on a gradient of the linear line.

4. The image evaluation method according to claim 1,
wherein the feature points correspond to an inferior margin of a pubis and an anorectal angle; and
wherein the index value corresponds to at least one of a distance between the inferior margin of the pubis and the anorectal angle, and a change over time of the distance.

5. The image evaluation method according to claim 4,
wherein the index value is calculated by applying an image tracking technique with respect to the feature points.

6. The image evaluation method according to any one of claims 1 to 5, further comprising:
performing estimation of severity of hypofunction of at least one of the lower urinary tract function and the pelvic floor function of the subject based on the index value; and
outputting data corresponding to a result of the estimation.

7. The image evaluation method according to any one of claims 1 to 6, further comprising:
performing selection of a method of training pelvic floor muscles based on the index value; and
outputting data corresponding to a result of the selection.

8. The image evaluation method according to any one of claims 1 to 7, further comprising:
performing comparison between the index value obtained at one time point and the index value obtained at another time point; and
outputting data corresponding to a result of the comparison.

9. An image evaluation device, comprising:
an interface configured to acquire image data corresponding to an ultrasonic image in which a pelvic region of a subject is captured; and
a processor configured to:
apply image processing with respect to the image data to extract multiple feature points associated with a prescribed portion; and
calculate an index value associated with at least one of a lower urinary tract function and a pelvic floor function of the subject based on the feature points.

10. A computer program adapted to be executed by a processor installed in an image evaluation device, the computer program being configured to, when executed, to cause the image evaluation device to:
receive image data corresponding to an ultrasonic image in which a pelvic region of a subject is captured;
apply image processing with respect to the image data to extract multiple feature points associated with a prescribed portion; and
calculate an index value associated with at least one of a lower urinary tract function and a pelvic floor function of the subject based on the feature points.

11. A non-transitory computer-readable medium having stored the computer program according to claim 10.
